# EUROPEAN PATENT APPLICATION

(11) **EP 2 573 084 A1**
(43) Date of publication of application: **27.03.2013**
(21) Application number: 11182287.0
(22) Date of filing: 22.09.2011
(51) Int. Cl.: C07D 413/14

(54) **Novel crystalline forms of rivaroxaban and processes for their preparation**

(71) Applicant: Enantia, S.L., 08028 Barcelona (ES)
(72) Inventor: Tesson, Nicolas, 08028 Barcelona (ES); Rafecas Jané, Llorenç, 08028 Barcelona (ES); Cárdenas Romaña, Lydia, 08028 Barcelona (ES)
(74) Representative: ZBM Patents

(57) **Abstract**

The present invention relates to novel crystalline forms of rivaroxaban, in particular, rivaroxaban of formula (I) in the crystalline form B1, the crystalline form B2, and crystalline form E. It also relates to a process of preparing said polymorphic forms and their conversion into other crystalline forms.

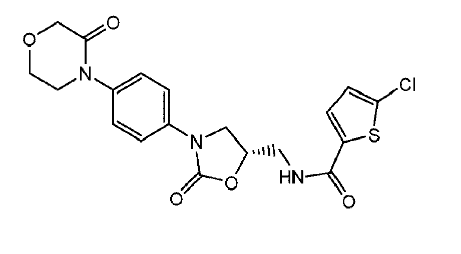

## Description

The present invention relates to novel crystalline forms of rivaroxaban. It also relates to a process of preparing said polymorphic forms and their conversion into other crystalline forms.

### BACKGROUND ART

Rivaroxaban is the generic name of compound 5-chloro-*N*{[(5*S*)-2-oxo-3-[4-(3-oxomorpholin-4-yl)phenyl]oxazolidin-5-yl]methyl}thiophene-2-carboxamide, the chemical structure of which is the following:

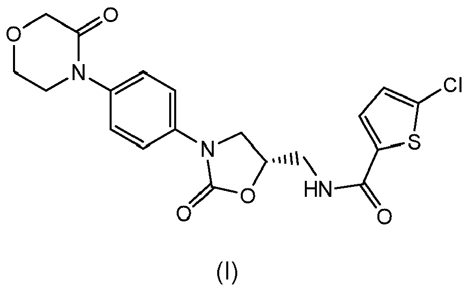

Rivaroxaban is currently used as anti-thrombotic agent. It was first disclosed in document WO 01/47919. The preparation of rivaroxaban as described in example 44 of this patent application is shown in the scheme below:

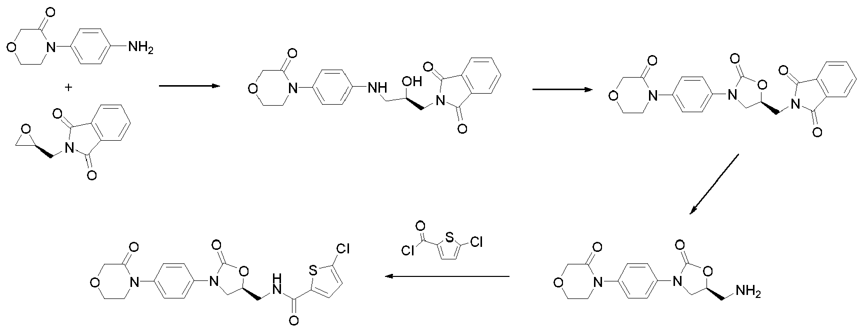

The above route of synthesis comprises the reaction of 4-(4-aminophenyl)-3-morpholinone and 2-[(2S)-2-oxiranylmethyl]-1H-isoindol-1,3(2H)-dione to yield an aminoalcohol, which is subsequently converted into an oxazolidinone. After deprotection of the amino group, the resulting compound is further reacted with 5-chlorothiophen-2-carbonyl chloride to yield rivaroxaban. The obtained rivaroxaban corresponds to a crystal modification which is designated as form I according to document WO 2007/039132 (filed by the same applicant).

Document WO 2007/039132 discloses further crystalline forms of rivaroxaban as well as an amorphous form of rivaroxaban. The disclosed polymorphic forms are referred to as: rivaroxaban form II (m.p. 203°C, transition point: 195°C; example 2); rivaroxaban form III (transition point: 127°C; example 3); rivaroxaban hydrate (containing about 4% of water; example 4); rivaroxaban NMP solvate (containing about 18.5% of N-methylpyrrolidone; example 5); and rivaroxaban inclusion compound with THF (containing about 5-7% of tetrahydrofuran, example 6).

The same document describes several processes for the preparation of rivaroxaban form II in example 2, which is more soluble than the rivaroxaban crystalline form I. These processes use large amounts of solvent (200-400 volumes with respect to the starting material), and/or require the evaporation of the solvent in a drying oven, and/or comprise working with the unstable amorphous form of rivaroxaban. Thus, these processes are difficult for industrialization and/or not easily reproducible.

WO 2007/039132 also describes the preparation of rivaroxaban form III in example 3. This process also uses a huge amount of solvent (832 total volumes with respect to the starting material) and, therefore, is not suitable for industrialization.

Another crystalline form of rivaroxaban is also described in document WO 2010/075631. This crystalline form, which is referred to as form APO-A is characterized by an X-ray diffraction pattern comprising a peak at 39.12 or 34.60 degrees two-theta. According to the description, this crystalline form provides for reduced residual organic solvent in the crystalline form when compared to other polymorphic forms of rivaroxaban.

Therefore, there is a need of having alternative processes for the preparation of rivaroxaban polymorphic forms, especially crystalline form II and crystalline form III, in particular of processes which are robust and easy to industrialize.

### SUMMARY OF THE INVENTION

Rivaroxaban form I as described in WO 01/47919 is the most stable form of rivaroxaban and is very insoluble in water and in most industrial solvents. Thus, the complete dissolution of this crystalline form requires large amounts of solvents, and should be avoided in an industrial process. The inventors have found that the slurrying of rivaroxaban form I in a mixture of an organic solvent and an acid furnish new crystal forms that can be used to obtain rivaroxaban form II and rivaroxaban form III (both as defined in WO 2007/039132) in a particularly effective process suitable for industrialization.

The process of the present invention overcomes the disadvantages of the prior art since it uses much lower amounts of solvent, preferably not higher than 20 volumes; the solvents and acids are industrially applicable and cheap; and the process shows a good reproducibility.

Additionally, the process of the invention renders rivaroxaban crystalline form II or rivaroxaban crystalline form III in high yields.

Therefore, an aspect of the present invention relates to a process for the preparation of rivaroxaban of formula (I) 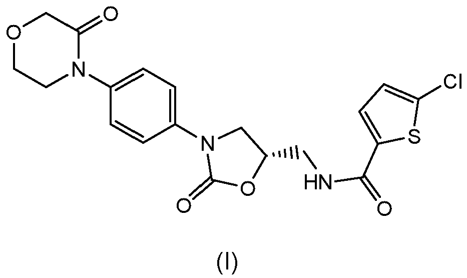 in the crystalline form II comprising:
a) slurrying rivaroxaban of formula (I) in a crystalline form selected from the crystalline form B2 and the crystalline form E in isopropanol; and
b) isolating rivaroxaban of formula (I) in the crystalline form II;
   wherein:
   the crystalline form II has an X-ray powder diffraction pattern comprising 26 angle values at 12.8, 17.7, 18.1, 18.4, 18.9, 19.9, 20.8, 21.6, 22.0, 22.9, 24.0, 26.0, 26.4, 26.6, 27.1, 27.5, 31.0, and 31.5 ± 0.1 measured in an X-ray diffractometer with Cu Kα radiation (1.5418 A);
   the crystalline form B2 has an X-ray powder diffraction pattern comprising 2θ angle values at 7.1, 14.2, 17.5, 19.8, 20.0, 21.4, 24.1, 24.5, 27.0, 28.7, 30.6, 39.8, 40.9, and 45.1 ± 0.1 measured in an X-ray diffractometer with Cu Kα radiation (1.5418 A); and
   the crystalline form E has an X-ray powder diffraction pattern comprising 2θ angle values at 14.5, 18.2, 19.2, 20.2, 20.8, 21.9, 24.7, 28.1, and 31.9 ± 0.1 measured in an X-ray diffractometer with Cu Kα radiation (1.5418 A).

Another aspect of the invention relates to rivaroxaban of formula (I) in the crystalline form B2, having an X-ray powder diffraction pattern comprising 2θ angle values at 7.1, 14.2, 17.5, 19.8, 20.0, 21.4, 24.1, 24.5, 27.0, 28.7, 30.6, 39.8, 40.9, and 45.1 ± 0.1 measured in an X-ray diffractometer with Cu Kα radiation (1.5418 A).

Another aspect of the invention relates to rivaroxaban of formula (I) in the crystalline form E, having an X-ray powder diffraction pattern comprising 2θ angle values at 14.5, 18.2, 19.2, 20.2, 20.8, 21.9, 24.7, 28.1, and 31.9 ± 0.1 measured in an X-ray diffractometer with Cu Kα radiation (1.5418 A).

Another aspect of the invention relates to a process for the preparation of rivaroxaban of formula (I) in the crystalline form III comprising drying rivaroxaban of formula (I) in the crystalline form B1 at a temperature from 40°C to 100°C; wherein:
the crystalline form III has an X-ray powder diffraction pattern comprising 2θ angle values at 19.2, 19.7, 19.9,23.3,23.8,24.3,28.3, and 31.2 ± 0.1 measured in an X-ray diffractometer with Cu Kα radiation (1.5418 A); and
the crystalline form B1 has an X-ray powder diffraction pattern comprising 2θ angle values at 7.1, 14.2, 17.7, 19.7, 19.8, 20.1, 21.5, 24.2, 24.6, 27.2, 28.8, 40.0, 40.9, and 45.4 ± 0.1 measured in an X-ray diffractometer with Cu Kα radiation (1.5418 A).

Another aspect of the invention relates to rivaroxaban of formula (I) in the crystalline form B1 having an X-ray powder diffraction pattern comprising 2θ angle values at 7.1, 14.2, 17.7, 19.7, 19.8, 20.1, 21.5, 24.2, 24.6, 27.2, 28.8, 40.0, 40.9, and 45.4 ± 0.1 measured in an X-ray diffractometer with Cu Kα radiation (1.5418 A).

Another aspect of the invention relates to a process for the preparation of rivaroxaban of formula (I) in the crystalline form B1 as defined above comprising:
(a) slurrying rivaroxaban of formula (I) in a crystalline form selected from the crystalline form B2 and the crystalline form E in water; and
(b) isolating the product obtained in step a);
   wherein the crystalline form B2 and the crystalline form E are as defined above.

Another aspect of the invention relates to a process for the preparation of rivaroxaban of formula (I) in the crystalline form B1 as defined above comprising washing rivaroxaban of formula (I) in the crystalline form B2 with water; wherein the crystalline form B2 is as defined above.

### BRIEF DESCRIPTION OF THE DRAWINGS

FIG. 1 shows the X-ray powder diffraction pattern (intensity (counts) vs. 2theta angle (°)) of rivaroxaban of formula (I) in the crystalline form B2.
FIG. 2 shows the FTIR spectrum of rivaroxaban of formula (I) in the crystalline form B2.
FIG. 3 shows the X-ray powder diffraction pattern (intensity (counts) vs. 2theta angle (°)) of rivaroxaban of formula (I) in the crystalline form E.
FIG. 4 shows the FTIR spectrum of rivaroxaban of formula (I) in the crystalline form E.
FIG. 5 shows the X-ray powder diffraction pattern (intensity (counts) vs. 2theta angle (°)) of rivaroxaban of formula (I) in the crystalline form B1.
FIG. 6 shows the FTIR spectrum of rivaroxaban of formula (I) in the crystalline form B1.

### DETAILED DESCRIPTION OF THE INVENTION

As mentioned above, the invention provides a process suitable for industrialization for the preparation of rivaroxaban crystalline form II and crystalline form III starting from new polymorphic forms which can be obtained from rivaroxaban crystalline form I.

Rivaroxaban form I is defined in WO 2007/039132 by having peak maxima at 4082, 4142, 4170, 4228, 4299, 4376, 4429, 4479, 4633, 4791, 4877, 4907, 5081, 5760, 5885, 6002, 6441, 6564, 8473, and 8833 in the near infrared (NIR) spectrogram. It is also characterized by having a melting point of 230°C. This crystalline form I has an X-ray powder diffraction pattern comprising 2θ angle values at 9.0, 12.0, 14.3, 16.5, 17.4, 18.0, 19.5, 19.9, 21.7, 22.5, 23.3, 24.1, 24.7, 25.6, 26.6, 30.1, and 31.8 ± 0.1 measured in an X-ray diffractometer with Cu Kα radiation (1.5418 A). Rivaroxaban form I may be obtained by the process described in WO 01/47919.

Rivaroxaban form II is defined in WO 2007/039132 by having peak maxima at 4086, 4228, 4418, 4457, 4634, 4905, 5846, 5911, 6026, 6081, and 6582 in the near infrared (NIR) spectrogram. This crystalline form II has an X-ray powder diffraction pattern comprising 2θ angle values at 12.8, 17.7, 18.1, 18.4, 18.9, 19.9, 20.8, 21.6, 22.0, 22.9, 24.0, 26.0, 26.4, 26.6, 27.1, 27.5, 31.0, and 31.5 ± 0.1 measured in an X-ray diffractometer with Cu Kα radiation (1.5418 A).

Rivaroxaban form III is defined in WO 2007/039132 by having peak maxima at 4080, 4218, 4329, 4398, 4606, 4891, 5066, 6022, and 6072 in the near infrared (NIR) spectrogram. The crystalline form III has an X-ray powder diffraction pattern comprising 2θ angle values at 19.2, 19.7, 19.9, 23.3, 23.8, 24.3, 28.3, and 31.2 ± 0.1 measured in an X-ray diffractometer with Cu Kα radiation (1.5418 A).

The crystalline form II may be obtained from an intermediate crystalline form B2 or E by the following steps:
a) slurrying rivaroxaban of formula (I) in a crystalline form selected from the crystalline form B2 and the crystalline form E in isopropanol; and
b) isolating rivaroxaban of formula (I) in the crystalline form II.

In a standard process, step a) is carried out at a temperature from -10°C to 40°C. In a preferred embodiment, the temperature is from -10 to 5°C, when the starting product is rivaroxaban of formula (I) in the crystalline form B2. In another preferred embodiment, the temperature is from -10°C to 40°C, more preferably from room temperature to 40°C, when the starting product is rivaroxaban of formula (I) in the crystalline form E.

For the purposes of the present invention, room temperature refers to a temperature from 20°C to 25°C.

The mixture is stirred for a suitable period of time, which can be from 1-22 hours, more preferably from 3 to 16 hours. Generally, the reaction time can be optimized depending on the reaction temperature.

Generally, the amount of isopropanol used in the reaction may be from 5 to 20 volumes with respect to the starting material. In a preferred embodiment, the amount of isopropanol is 10 to 20 volumes. Thus, the amount of solvent used in this step is very significantly reduced in comparison with the prior art processes.

In a preferred embodiment, the process further comprises the additional step of seeding the slurry obtained in isopropanol with a previously obtained rivaroxaban of formula (I) in the crystalline form II.

The isolation of the crystalline form II can be normally performed by any suitable industrial isolation method, such as filtration or centrifugation.

In a preferred embodiment, the isolation of the crystalline form II comprises filtration, which can be performed, for instance, by using a sintered funnel of a suitable porosity. In another preferred embodiment, after filtration, the product is washed with isopropanol.

In a particular embodiment, the process further comprises an additional step of drying the product isolated in step b). Drying may be achieved optionally under vacuum at a suitable temperature, preferably at room temperature.

The intermediate crystalline forms B2 and E can be obtained from the crystalline form I by slurrying the later crystalline form in a mixture of an organic solvent and an acid. Depending on the acid used, either form B2 or form E is obtained.

Thus, the crystalline form B2 may be obtained by using an acid other than HNO₃. The crystalline form E is obtained by using HNO₃.

Thus, in a preferred embodiment, in the preparation of rivaroxaban of formula (I) in the crystalline form II, the intermediate rivaroxaban crystalline form B2 is obtained by previously:
(i) slurrying rivaroxaban of formula (I) in the crystalline form I in a mixture of an organic solvent and an acid other than HNO₃; and
(ii) isolating the product obtained in step (i) to obtain rivaroxaban of formula (I) in the crystalline form B2.

In another preferred embodiment, in the preparation of rivaroxaban of formula (I) in the crystalline form II, the intermediate rivaroxaban crystalline form E is obtained by previously:
(i) slurrying rivaroxaban of formula (I) in the crystalline form I in a mixture of an organic solvent and HNO₃; and
(ii) isolating the product obtained in step (i) to obtain rivaroxaban of formula (I) in the crystalline form E.

As it will be seen in the examples, the above processes take place in high yields.

Generally, the organic solvent may be a (C₁-C₆)alcohol such as methanol, ethanol or isopropanol; a (C₃-C₈)ketone, such as acetone, methylethylketone or methylisobutylketone; a (C₁-C₆)alkyl(C₁-C₆)ester, such as ethyl acetate; a (C₃-C₆)amide, such as dimethylformamide, dimethylacetamide or N-methylpyrrolidone; a (C₃-C₆)ether such as tetrahydrofuran or dioxane; or a (C₆-C₉)aromatic solvent, such as toluene or xylene. Preferably, the reaction is carried out in a (C₁-C₆)alcohol or a (C₆-C₉)aromatic solvent. More preferably, the reaction is carried out in methanol or toluene.

In a preferred embodiment, the acid used in the above processes is added at low temperature, more preferably from 0° to 5°C. This low temperature may be optionally maintained for a short period of time, e.g., 10 min. After that, the temperature is preferably raised to room temperature and left stand for a suitable period of time, which can be from 1-3 hours, more preferably 1.5 hours.

The acid used in the preparation of the crystalline form B2 may be organic or inorganic with the proviso that it is other than HNO₃. Examples of suitable inorganic acids include HCl and H₃PO₄. Examples of suitable organic acids include formic acid and acetic acid. In a preferred embodiment, the acid is an inorganic acid other than HNO₃. In a more preferred embodiment, the acid is HCl. In an even more preferred embodiment, the acid is concentrated aqueous HCl (generally 35-37%).

In another preferred embodiment, the HNO₃ used for the preparation of the crystalline form E is aqueous HNO_{3.} By way of example 5M, 8M, 10M, or 65% aqueous HNO₃ solutions may be used for the preparation of the crystalline form E.

In a standard process, the amount of solvent used in the reaction above for the preparation of both the crystalline forms B2 or E may be from 5 to 15 volumes with respect to the starting material, and the amount of acid may be from 1 to 15 volumes, preferably from 2.5 to 15 volumes, more preferably from 5 to 15 volumes, with respect to the starting material. Thus, the amount of solvent used in this step is very significantly reduced in comparison with the prior art processes.

In another preferred embodiment, the processes for the preparation of the crystalline forms B2 and E comprise the additional step of seeding the slurry obtained in the mixture organic solvent/acid with the corresponding crystalline form (B2 or E).

The isolation of the crystalline form B2 or crystalline form E can be normally performed by any suitable industrial isolation method, such as filtration or centrifugation. In a preferred embodiment, the isolation comprises filtering the product obtained, for instance, the filtration can be performed by using a sintered funnel of a suitable porosity; and optionally washing with an organic solvent. Generally, the organic solvent used for the washing can be an organic solvent as described above. Preferably, the solvent used for the washing is a (C₁-C₆)alcohol or a (C₆-C₉)aromatic solvent, more preferably methanol or toluene. More preferably, the solvent used for the washing is the same solvent used for slurrying rivaroxaban of formula (I) in the crystalline form I.

In another preferred embodiment, the intermediate crystalline form B2 or E obtained is dried. In a standard process, Drying may be achieved optionally under vacuum at a suitable temperature, preferably at a temperature from room temperature to 40°C.

In a preferred embodiment, rivaroxaban of formula (I) in the crystalline form B2 is isolated and dried without any washing step. In another preferred embodiment, rivaroxaban of formula (I) in the crystalline form E is dried after a washing step with methanol or toluene.

The most adequate conditions for carrying out the processes of the present invention vary depending on the parameters considered by the expert in the art. These can be easily determined by said skilled person in the art by routine tests and with the help of the teachings of the examples given in this description.

A process for the preparation of rivaroxaban of formula (I) in the crystalline form III also forms part of the invention. This process comprises drying rivaroxaban of formula (I) in a crystalline form B1 at a temperature from 40°C to 100°C, preferably from 40°C to 70°C, and more preferably at 40°C. In a preferred embodiment, the drying is carried out under vacuum. Generally, the drying time can be optimized depending on the drying temperature.

In a preferred embodiment, the intermediate rivaroxaban crystalline form B1 used in the above process is obtained by previously:
(a) slurrying rivaroxaban of formula (I) in a crystalline form selected from the crystalline form B2 and the crystalline form E in water; and
(b) isolating the product obtained in step (a) to obtain rivaroxaban of formula (I) in the crystalline form B1.

In a standard process, the amount of water used in the reaction may be from 5 to 20 volumes with respect to the starting material, although it can also be performed in higher amounts such as 30 volumes.

In a preferred embodiment, this step is carried out at a temperature from 0° to room temperature, more preferably, at room temperature. The mixture is stirred for a suitable period of time, which can be from 1-22 hours, more preferably from 3 to 16 hours.

The isolation of the crystalline form B1 can be normally performed by any suitable industrial isolation method, such as filtration or centrifugation. In a preferred embodiment, the isolation comprises filtration, which can be performed, for instance, by using a sintered funnel of a suitable porosity.

In another preferred embodiment, after filtration, the product is washed with water. After washing, the product can be dried optionally under vacuum at a suitable temperature, preferably at a temperature lower than 35°C, more preferably at room temperature.

Alternatively, the intermediate rivaroxaban crystalline form B1 may be directly obtained by washing rivaroxaban of formula (I) in the crystalline form B2 with water. This process forms also part of the present invention.

Generally, the amount of water used for the washing may be from 2 to 15 volumes with respect to the starting material. After washing, the product can be dried optionally under vacuum at a suitable temperature, preferably at a temperature lower than 35°C, more preferably at room temperature.

In a preferred embodiment, in the preparation of rivaroxaban of formula (I) in the crystalline form III, the intermediate rivaroxaban crystalline form B2, and which is subsequently slurried in or washed with water, is obtained by previously:
(i) slurrying rivaroxaban of formula (I) in the crystalline form I in a mixture of an organic solvent and an acid other than HNO₃; and
(ii) isolating the product obtained in step (i) to obtain rivaroxaban of formula (I) in the crystalline form B2.

The step (i) above relating to the preparation of the crystalline form B2 to be used in the preparation of form III is carried out in the same conditions and preferences as the ones already described in the corresponding embodiment relating to the preparation of the crystalline form B2 to be used in the preparation of the crystalline form II.

The isolation of the crystalline form B2 (step (ii) above) can be normally performed by any suitable industrial isolation method, such as filtration or centrifugation as previously mentioned. When the crystalline form B2 is subsequently slurried in or washed with water to yield B1, it is not necessary washing B2 with an organic solvent nor drying it, which is advantageous in an industrial process.

In another preferred embodiment, in the preparation of rivaroxaban of formula (I) in the crystalline form III, the intermediate rivaroxaban crystalline form E is obtained by previously:
(i) slurrying rivaroxaban of formula (I) in the crystalline form I in a mixture of an organic solvent and HNO₃; and
(ii) isolating the product obtained in step (i) to obtain rivaroxaban of formula (I) in the crystalline form E.

The step (i) above relating to the preparation of the crystalline form E to be used in the preparation of form III is carried out in the same conditions and preferences as the ones already described in the corresponding embodiment relating to the preparation of the crystalline form E to be used in the preparation of the crystalline form II.

The isolation of the crystalline form E (step (ii) above) can be normally performed by any suitable industrial isolation method, such as filtration or centrifugation as previously mentioned. When the crystalline form E is subsequently slurried in water to yield B1, it is not necessary drying E, which is advantageous in an industrial process.

The crystalline forms B1, B2, and E of rivaroxaban also form part of the invention and have been characterized by several techniques such as proton nuclear magnetic resonance (¹H-NMR), Fourier Transform Infrared (FTIR), Differential Scanning Calorimetry (DSC), Thermogravimetric (TGA), X-ray powder diffraction (XRPD).

The crystalline forms B1 and B2 are hydrates having similar XRPD patterns. However, the crystalline form B1 contains a lower amount of water in comparison with the crystalline form B2. The crystalline form E appears to be a nitric acid solvate.

Different crystalline forms can have different characteristics, and offer certain advantages, for example with regard to stability, solubility or bioavailability, which allows for improving the characteristics of the pharmaceutical formulations of the active ingredients, since some forms are more adequate for one type of formulation, and other forms for other different formulations. Furthermore, depending on the therapeutic indications, one or another pharmaceutical formulation may be preferred.

For example, rivaroxaban of formula (I) in the crystalline form B1 and B2 are stable crystalline forms in the conditions of storage (no modifications were observed by XRPD after several months of storage at room temperature). Furthermore, the crystalline forms B1 and B2 (B2 is converted to B1 in water) show a better solubility in water than the crystalline stable form I. The crystal form E is a very stable crystal form, which allows an easy manipulation for the preparation of the crystalline forms B1, II and III (no modifications observed by XRPD after several months of storage at room temperature).

Moreover, the crystalline forms B1, B2, and E forms are also useful intermediates in the preparation of other crystalline forms, in particular, form II or form III by robust processes, which are suitable for industrialization and take place in high yields.

In a preferred embodiment, rivaroxaban of formula (I) in the crystalline form B1 shows an X-ray powder diffraction pattern comprising 2θ angle values at 7.1, 12.3, 14.2, 17.7, 17.9, 19.7, 19.8, 20.1, 20.6, 21.5, 24.2, 24.6, 26.2, 27.2, 28.8, 30.1, 31.7, 33.6, 37.3, 37.8, 40.0, 40.9, and 45.4 ± 0.1 measured in an X-ray diffractometer with Cu Kα radiation (1.5418 A).

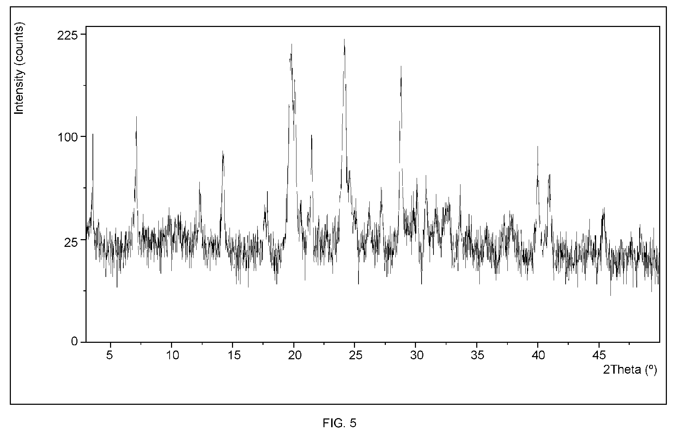

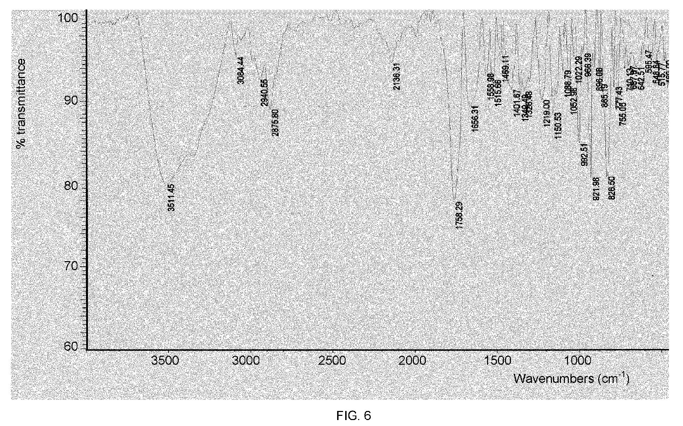

In another preferred embodiment, the crystalline form B1 shows an X-ray powder diffraction pattern comprising 2θ angle values substantially according to Table 3. In another preferred embodiment, the crystalline form B1 shows an X-ray powder diffraction pattern substantially according to FIG. 5. In another preferred embodiment, the crystalline form B1 shows a FTIR spectrum substantially according to FIG. 6.

Surprisingly, the crystalline form B1 is more soluble in water than form I, as it is also the case of the crystalline form II. As rivaroxaban belongs to the Biopharmaceutics Classification System (BCS) class II, an improvement of the solubility should increase its bioavailability. In addition, the inventors have found that the crystalline form B1 is more stable than the crystalline form II. Thus, rivaroxaban hydrate form B1 remained stable in water at room temperature even after 8h, while a slurrying of rivaroxaban form II in water at room temperature, rendered a partial transformation in the stable form rivaroxaban form I after 1 h.

The present invention also relates to rivaroxaban of formula (I) in the crystalline form B1 for use in the treatment and/or prophylaxis of diseases, preferably thromboembolic diseases and/or thromboembolic complications.

It forms also part of the invention the use of rivaroxaban of formula (I) in the crystalline form B1 for the manufacture of a medicament for the treatment and/or prophylaxis of diseases, preferably thromboembolic diseases and/or thromboembolic complications.

The present invention also relates to a method for the treatment and/or prophylaxis of diseases, preferably thromboembolic diseases and/or thromboembolic complications, comprising administering a pharmaceutically effective amount of rivaroxaban of formula (I) in the crystalline form B1, together with pharmaceutically acceptable excipients or carriers, in a subject in need thereof, including a human.

Further, the invention also relates to rivaroxaban of formula (I) in the intermediate crystalline form B2. In a preferred embodiment, the crystalline form B2 shows an X-ray powder diffraction pattern comprising 2θ angle values at 7.1, 14.2, 17.5, 17.8, 19.8, 20.0, 21.4, 24.1, 24.5, 24.9, 26.2, 27.0, 28.7, 30.1, 30.6, 32.4, 39.8, 40.9, and 45.1 ± 0.1 measured in an X-ray diffractometer with Cu Kα radiation (1.5418 A).

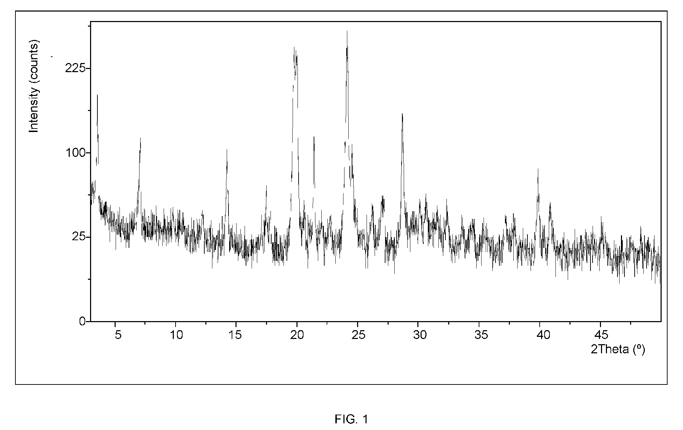

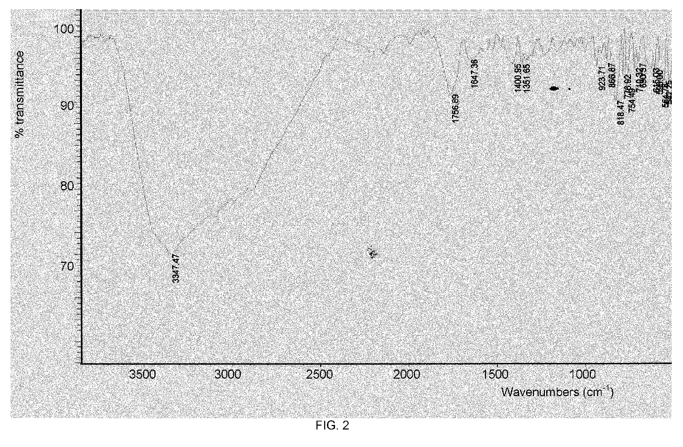

In another preferred embodiment, the crystalline form B2 shows an X-ray powder diffraction pattern comprising 2θ angle values substantially according to Table 1. In another preferred embodiment, the crystalline form B2 shows an X-ray powder diffraction pattern substantially according to FIG. 1. In another preferred embodiment, the crystalline form B2 shows a FTIR spectrum substantially according to FIG. 2.

Additionally, the invention also relates to rivaroxaban of formula (I) in the intermediate crystalline form E. In a preferred embodiment, the crystalline form E shows an X-ray powder diffraction pattern comprising 2θ angle values at 14.5, 18.2, 18.4, 19.2, 19.6, 20.2, 20.8, 21.9, 23.2, 23.7, 23.9, 24.7, 26.1, 26.4, 28.1, 28.4, 29.3, 30.3, 31.9, 33.8, 34.2, 39.9, and 41.1 ± 0.1 measured in an X-ray diffractometer with Cu Kα radiation (1.5418 A).

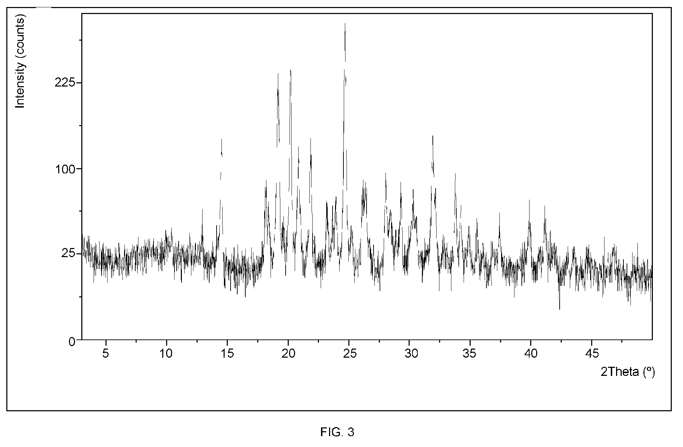

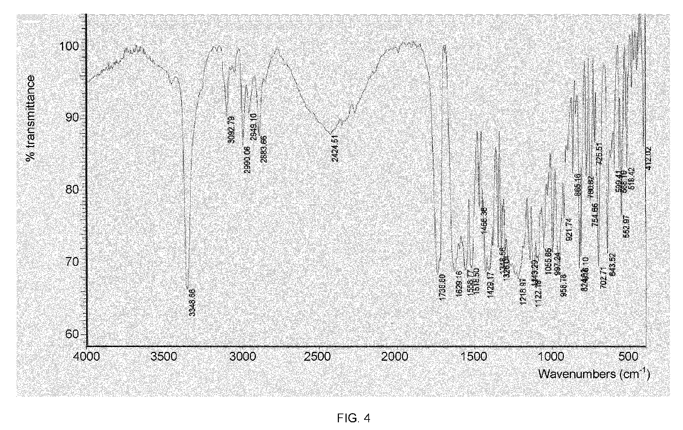

In another preferred embodiment, the crystalline form E shows an X-ray powder diffraction pattern comprising 2θ angle values substantially according to Table 2. In another preferred embodiment, the crystalline form E shows an X-ray powder diffraction pattern substantially according to FIG. 3. In another preferred embodiment, the crystalline form E shows a FTIR spectrum substantially according to FIG. 4. In another preferred embodiment, the crystalline form E has a melting point of 148.2 °C.

The present invention also provides processes for preparing the novel crystalline forms of the invention B1, B2 and E.

The crystalline form B1 may be prepared starting from the crystalline form I; the crystalline forms B2 or E; or from the crystalline form III.

Thus, the present invention provides a process for the preparation of the crystalline form B1 comprising:
a) slurrying rivaroxaban of formula (I) in a crystalline form selected from the crystalline form B2 and the crystalline form E in water; and
b) isolating the product obtained in step a).

The latter process for the preparation of form B1 above is carried out in the same conditions and preferences as the ones already described in the corresponding embodiment relating to the preparation of the crystalline form B1 to be used in the preparation of the crystalline form III.

The present invention also provides a process for the preparation of rivaroxaban of formula (I) in the crystalline form B1 comprising washing rivaroxaban of formula (I) in the crystalline form B2 with water. This washing step is carried out in the same conditions and preferences as the ones already described in the corresponding embodiment of the process for the preparation of rivaroxaban of formula (I) in the crystalline form III.

In a particular embodiment, the crystalline form B1, either obtained by slurrying rivaroxaban form B2 or E in water or by washing rivaroxaban form B2 with water, is dried after its isolation. Drying may be achieved optionally under vacuum at a suitable temperature, preferably at a temperature lower than 35°C, more preferably at room temperature.

In a preferred embodiment, in the preparation of rivaroxaban of formula (I) in the crystalline form B1, the intermediate rivaroxaban crystalline form B2, and which is subsequently slurried in or washed with water, is obtained by previously:
(i) slurrying rivaroxaban of formula (I) in the crystalline form I in a mixture of an organic solvent and HCl; and
(ii) isolating the product obtained in step (i) to obtain rivaroxaban of formula (I) in the crystalline form B2.

In another preferred embodiment, in the preparation of rivaroxaban of formula (I) in the crystalline form B1, the intermediate rivaroxaban crystalline form E is obtained by previously:
(i) slurrying rivaroxaban of formula (I) in the crystalline form I in a mixture of an organic solvent and HNO₃; and
(ii) isolating the product obtained in step (i) to obtain rivaroxaban of formula (I) in the crystalline form E.

The latter two embodiments relating to the preparation of the crystalline forms B2 or E to be used in the preparation of form B1 above are carried out in the same conditions and preferences as the ones already described in the corresponding embodiments relating to the preparation of the crystalline forms B2 or E to be used in the preparation of the crystalline form II. Alternatively, the present invention also provides a process for the preparation of the crystalline form B1 comprising submitting the crystalline form III under humidity conditions, preferably in an atmosphere having at least 49% humidity, more preferably, at a temperature from 20-30°C and humidity of 49-100%.

Further, the crystalline form B2 and form E may be prepared starting from the crystalline form I.

Thus, the present invention provides a process for the preparation of the crystalline form B2, which comprises:
a) slurrying rivaroxaban of formula (I) in the crystalline form I in a mixture of an organic solvent and HCl; and
b) isolating the compound obtained in step a).

The latter process for the preparation of form B2 above is carried out in the same conditions and preferences as the ones already described in the corresponding embodiment relating to the preparation of the crystalline form B2 to be used in the preparation of the crystalline form II.

Further, the present invention also provides a process for the preparation of the nitric acid solvate form E, which comprises:
a) slurrying rivaroxaban of formula (I) in the crystalline form I in a mixture of an organic solvent and HNO₃; and
b) isolating the compound obtained in step a).

The latter process for the preparation of form E above is carried out in the same conditions and preferences as the ones already described in the corresponding embodiment relating to the preparation of the crystalline form E to be used in the preparation of the crystalline form II.

Throughout the description and claims the word "comprise" and variations of the word, are not intended to exclude other technical features, additives, components, or steps. Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments described herein.

### EXAMPLES

Proton nuclear magnetic resonance (¹H-NMR) analyses were recorded in deuterated dimethyl sulfoxide (d6-DMSO) in a Varian Mercury 400 spectrometer, equipped with a broadband probe ATB 1 H/19F/X of 5 mm. Spectra were acquired dissolving 5-10 mg of sample in 0.6 mL of deuterated solvent.

Fourier Transform Infrared (FTIR) spectra were recorded using a Thermo Nicolet Nexus 870 FT-IR, equipped with a beamsplitter KBr system, a 35 mW He-Ne laser as the excitation source and a DTGS KBr detector. The spectrum was acquired in 32 scans at a resolution of 4 cm⁻¹.

Differential Scanning Calorimetry (DSC) analyses were recorded with a Mettler DSC822^{e} Samples of the products were weighed into 40 µL aluminium crucible with a pinhole lid and was heated, under nitrogen (50 mL/min), at 10°C/min from 30 to 300 °C.

Thermogravimetric (TGA) analyses were recorded in a thermogravimetric analyzer Mettler TGA/SDTA851^{e}. Samples of the products were weighed into a 70 µL alumina crucible with a pinhole lid and was heated at 10 °C/min from 30 to 300 °C, under nitrogen (50 mL/min).

X-ray powder diffraction (XRPD) analyses were performed using a PANalytical X'Pert diffractometer with Cu K_{α} radiation in Bragg-Brentano geometry. The system was equipped with a monodimensional, real time multiple strip detector. The diffractograms were recorded from 3° to 50° (2θ) at a scan rate of 17.8° per minute.

Karl Fischer analyses were recorded with a Metrohm 787 KF Trinito.

### Examples 1-4: Preparation of 5-chloro-N-{[(55)-2-oxo-3-[4-(3-oxomorpholin-4-yl)phenyl]oxazolidin-5-yl]methyl}thiophene-2-carboxamide hydrate form B2

### Example 1: Slurrying in MeOH in the presence of concentrated HCl from rivaroxaban form I

To a round-bottom flask equipped with magnetic stirrer containing rivaroxaban form I (150 mg, 0.34 mmol) was added methanol (2.3 mL, 15 vol.) and was stirred for a few minutes. Then, hydrochloric acid 37% (2.3 mL, 15 vol.) was added dropwise at 0°C. The resultant suspension was stirred at room temperature for 1.5 hours. The solid was filtered with a sintered funnel (porosity 4) and dried under vacuum at room temperature to give rivaroxaban of formula (I) in the crystalline form B2 as a pale yellow solid (145 mg, 89% approximated yield).

### Example 2: Slurrying in MeOH in the presence of concentrated HCl from rivaroxaban form I

To a round-bottom flask equipped with magnetic stirrer containing rivaroxaban form I (5.00 g, 11.47 mmol) was added methanol (75 mL, 15 vol.) and was stirred for a few minutes. Then, hydrochloric acid 37% (75 mL, 15 vol.) was added dropwise at 0°C. The resultant suspension was seeded with rivaroxaban of formula (I) in the crystalline form B2 and was stirred at room temperature for 1.5 hours. The solid was filtered with a sintered funnel (porosity 3) and dried under vacuum at room temperature to give rivaroxaban of formula (I) in the crystalline form B2 as an off-white solid (4.95 g, 92% approximated yield).

### Example 3: Slurrying in MeOH in the presence of concentrated HCl from rivaroxaban form I

To a round-bottom flask equipped with magnetic stirrer containing rivaroxaban form I (345 mg, 0.79 mmol) was added methanol (1.7 mL, 5 vol.) and was stirred for a few minutes at 0°C. Then, hydrochloric acid 37% (3.45 mL, 10 vol.) was added dropwise at 0°C. The resultant suspension was stirred at room temperature for 1.5 hours. The solid was filtered with a sintered funnel (porosity 4) and dried under vacuum at room temperature to give rivaroxaban of formula (I) in the crystalline form B2 as an off-white solid (330 mg, 88% yield).

### Example 4: Slurrying in toluene in the presence of concentrated HCl from rivaroxaban form I

To a round-bottom flask equipped with magnetic stirrer containing rivaroxaban form I (20 mg, 0.05 mmol) was added toluene (0.3 mL, 15 vol.) and was stirred for a few minutes at 0°C. Then, hydrochloric acid 37% (0.02 mL, 1 vol) was added dropwise at 0°C. The resultant two phases were stirred at room temperature for 1.5 hours. The solid was filtered with a sintered funnel (porosity 4) and dried under vacuum at room temperature to give rivaroxaban of formula (I) in the crystalline form B2 as an off-white solid (20 mg, 93% approximated yield).

¹H NMR (d6-DMSO, 400 MHz): δ = 8.98 (t, J = 5.9 Hz, 1 H); 7.69 (d, J = 4.0 Hz, 1 H); 7.58-7.53 (m, 2H); 7.42-7.36 (m, 2H); 7.19 (d, J = 4.0 Hz, 1 H); 4.90-4.79 (m, 1 H); 4.19 (s, 2H); 4.23-4.14 (m, 1 H); 4.00-3.94 (m, 2H); 3.85 (dd, J = 6.2 and 9.0 Hz, 1 H); 3.75-3.67 (m, 2H); 3.60 (t, J = 5.5 Hz, 2H).

IR (KBr): absorption bands at 3348 (s), 1757 (m), 1647 (w), 1401 (w), 1352 (w), 924 (w), 819 (m) cm⁻¹.

DSC: Rivaroxaban of formula (I) in the crystalline form B2 is characterized for two endothermic intense peaks corresponding to the water loss at an onset 71.95°C (fusion enthalpy -191.51 J/g) and the melting point at an onset 228.78 °C (fusion enthalpy -101.13 J/g) and two exothermic sharp peaks corresponding to a polymorphic transformations at an onset 139.84 °C (formation enthalpy 12.50 J/g) and at an onset 177.72 °C (formation enthalpy 7.20 J/g), measured by DSC analysis (10 °C/min).

TGA: The TG analysis of rivaroxaban of formula (I) in the crystalline form B2 shows a weight loss at temperatures lower than the melting point. It corresponds to water loss (9.8%). A TGA of another batch of the same crystalline form gave results a little different (loss of water 8.4%) for the possibility of a water loss depending on the atmospheric conditions (but this loss of water in these conditions does not seem change the crystal form). XRPD: The XRPD pattern of rivaroxaban of formula (I) in the crystalline form B2 is shown in FIG. 1. Table 1 shows the position (2theta angle (°)) and relative intensity (%) of the main peaks of the XRPD spectrum of FIG. 1.

**Table 1**

| Position [°2Th.] | Relative intensity [%] |
|---|---|
| 3.6 | 51 |
| 7.1 | 38 |
| 12.2 | 8 |
| 14.2 | 33 |
| 17.5 | 16 |
| 17.8 | 11 |
| 19.8 | 98 |
| 20.0 | 100 |
| 20.6 | 9 |
| 21.4 | 39 |
| 22.0 | 7 |
| 22.7 | 8 |
| 24.1 | 98 |
| 24.5 | 35 |
| 24.9 | 17 |
| 26.2 | 11 |
| 27.0 | 14 |
| 28.7 | 56 |
| 30.1 | 11 |
| 30.6 | 11 |
| 31.5 | 7 |
| 32.4 | 10 |
| 33.6 | 5 |
| 34.5 | 4 |
| 35.5 | 3 |
| 37.2 | 7 |
| 37.8 | 5 |
| 38.6 | 6 |
| 39.8 | 22 |
| 40.9 | 12 |
| 45.1 | 5 |
| 47.5 | 3 |
| 48.3 | 5 |

### Examples 5-8: Preparation of 5-chloro-N-{[(5S)-2-oxo-3-[4-(3-oxomorpholin-4-yl)phenyl]oxazolidin-5-yl]methyl}thiophene-2-carboxamide nitric acid solvate form E

### Example 5: Slurrying in MeOH from rivaroxaban form I in the presence of HNO₃ 65%

To an assay tube equipped with magnetic stirrer containing rivaroxaban form I (20 mg, 0.05 mmol) was added methanol (0.3 mL, 15 vol.). The resultant suspension was stirred at 0-5°C for a few minutes. Then, nitric acid 65% (0.3 mL, 15 vol.) was added dropwise at 0-5°C. The mixture was stirred at 0-5°C for 10 minutes and at room temperature for 1.5 hours. The solid was filtered with a sintered funnel (porosity 4) and dried under vacuum at room temperature to give rivaroxaban of formula (I) in the crystalline form E as a white solid (18 mg, 78% yield).

### Example 6: Slurrying in MeOH from rivaroxaban form I in the presence of HNO₃ 65%

To an assay tube equipped with magnetic stirrer containing rivaroxaban form I (300 mg, 0.69 mmol) was added methanol (4.5 mL, 15 vol.). The resultant suspension was stirred at 0-5°C for a few minutes and was seeded with rivaroxaban of formula (I) in the crystalline form E. Then, nitric acid 65% (1.5 mL, 5 vol.) was added dropwise at 0-5°C. The mixture was stirred at 0-5°C for 10 minutes and at room temperature for 1.5 hours. The solid was filtered with a sintered funnel (porosity 4), washed with methanol (2 x 1.5 mL) and dried under vacuum at room temperature to give rivaroxaban of formula (I) in the crystalline form E as a white solid (329 mg, 96% yield).

### Example 7: Slurrying in MeOH from rivaroxaban form I in the presence of a 8M aqueous HNO₃ solution

To an assay tube equipped with magnetic stirrer containing rivaroxaban form I (399 mg, 0.92 mmol) was added methanol (4 mL, 10 vol.). The resultant suspension was stirred at 0-5°C for a few minutes and was seeded with rivaroxaban of formula (I) in the crystalline form E). Then, nitric acid 8M aqueous solution (2 mL, 5 vol.) was added dropwise at 0-5°C. The mixture was stirred at 0-5°C for 10 minutes and at room temperature for 1.5 hours. The solid was filtered with a sintered funnel (porosity 4), washed with methanol (2 x 1 mL) and dried under vacuum at room temperature to give rivaroxaban of formula (I) in the crystalline form E as a white solid (435 mg, 95% yield).

### Example 8: Slurrying in toluene from rivaroxaban form I in the presence of a 5M aqueous HNO₃ solution

To an assay tube equipped with magnetic stirrer containing rivaroxaban form I (100 mg, 0.23 mmol) was added toluene (1.5 mL, 15 vol.). The resultant suspension was stirred at 0-5°C for a few minutes and was seeded with rivaroxaban of formula (I) in the crystalline form E). Then, nitric acid 10M aqueous solution (0.25 mL, 2.5 vol.) was added at 0-5°C. The mixture was stirred at 0-5°C for 10 minutes and at room temperature for 1.5 hours. The solid was filtered with a sintered funnel (porosity 4), washed with toluene (2 x 0.5 mL) and dried under vacuum at room temperature to give rivaroxaban of formula (I) in the crystalline form E as a white solid (99 mg, 86% yield).

¹H NMR (d6-DMSO, 400 MHz): δ = 8.96 (t, J = 5.8 Hz, 1 H); 7.68 (d, J = 3.9 Hz, 1 H); 7.59-7.52 (m, 2H); 7.43-7.37 (m, 2H); 7.19 (d, J = 3.9 Hz, 1 H); 7.29-6.91 (br. s, 2H); 4.90-4.78 (m, 1 H); 4.24-4.14 (m, 1 H); 4.19 (s, 2H); 4.00-3.92 (m, 2H); 3.85 (dd, J = 6.2 and 9.0 Hz, 1 H); 3.75-3.67 (m, 2H); 3.60 (t, J = 5.5 Hz, 2H).

IR (KBr): absorption bands at 3349 (s), 3093 (w), 2990 (w), 2949 (w), 2884 (w), 1739 (s), 1629 (s), 1559 (s), 1519 (s), 1429 (s), 1122 (s), 959 (s), 825 (s), 703 (s), 644 (s), 553 (m) cm⁻¹.

DSC: Rivaroxaban of formula (I) in the crystalline form E is characterized by an endothermic peak corresponding to the melting point with an onset at 148.21 °C (fusion enthalpy -19.86 J/g) followed by an exothermic sharp peak corresponding to a decomposition (decomposition enthalpy 46.95 J/g), measured by DSC analysis (10 °C/min)

TGA: The TG analysis of rivaroxaban of formula (I) in the crystalline form E shows a weight loss at temperatures between the melting point and the decomposition of the sample. It corresponds to HNO₃ loss (8.9 %) but it is altered by the decomposition of RVB (the loss of 1 HN03 molecule is supposed to be 12.6 %).

KF: Rivaroxaban of formula (I) in the crystalline form E was dissolved in a mixture of acetonitrile with 1 % of dimethylformamide (20 mg/4 mL). Three 0.79 mg samples of this solution were analyzed using the following reactants: Hydranal-Composite 5 (Riedel de Haën Ref. 34805), Hydranal Methanol Rapid (Riedel de Haën Ref. 37817) and Hydranal Water Standard 1.0 (Riedel de Haen Ref. 34828 used to calculate the factor). The KF analysis of rivaroxaban of formula (I) in the crystalline form E shows 0.06 % water. This is not a hydrate.

XRPD: The XRPD pattern of rivaroxaban of formula (I) in the crystalline form E is shown in FIG. 3. Table 2 shows the position (2theta angle (°)) and relative intensity (%) of the main peaks of the XRPD spectrum of FIG. 3.

**Table 2**

| Position [°2Th.] | Relative intensity [%] |
|---|---|
| 12.9 | 6 |
| 14.5 | 38 |
| 18.2 | 20 |
| 18.4 | 19 |
| 19.2 | 62 |
| 19.6 | 14 |
| 20.2 | 72 |
| 20.8 | 29 |
| 21.9 | 38 |
| 23.2 | 11 |
| 23.7 | 19 |
| 23.9 | 14 |
| 24.7 | 100 |
| 25.2 | 7 |
| 26.1 | 18 |
| 26.4 | 17 |
| 28.1 | 22 |
| 28.4 | 11 |
| 28.9 | 9 |
| 29.3 | 18 |
| 30.3 | 14 |
| 31.9 | 33 |
| 33.8 | 19 |
| 34.2 | 11 |
| 34.9 | 6 |
| 35.6 | 7 |
| 36.0 | 8 |
| 37.4 | 9 |
| 39.1 | 8 |
| 40.0 | 12 |
| 40.7 | 4 |
| 41.1 | 15 |
| 41.8 | 5 |
| 43.5 | 4 |
| 44.7 | 3 |
| 46.8 | 5 |

TITRATION: A suspension of rivaroxaban of formula (I) in the crystalline form E (100 mg, 0.20 mmol) in water (5 mL) was stirred at room temperature for 3 hours. The resultant suspension was titrated with a 0.1 M NaOH aq. solution to determine the amount of HNO₃ in rivaroxaban of formula (I) in the crystalline form E. The amount of 0.1 M NaOH aq. solution used in the analysis corresponds with a rivaroxaban nitric acid solvate with 1:1 stoichiometry.

### Examples 9-11: Preparation of 5-chloro-N-{[(5S)-2-oxo-3-[4-(3-oxomorpholin-4-yl)phenyl]oxazolidin-5-yl]methyl}thiophene-2-carboxamide hydrate form B1

### Example 9: Humidity atmosphere from rivaroxaban form III

To a crystallizer containing rivaroxaban form III (1.37 g, 3.14 mmol) was submitted at atmosphere conditions (temperature 24.2-27.2 °C and humidity 49-65 %) over a period of 1 day. Rivaroxaban hydrate form B1 was obtained as a white solid (quantitative yield).

### Example 10: Slurrying in water from rivaroxaban form B2

To a round-bottom flask equipped with magnetic stirrer containing rivaroxaban of formula (I) in the crystalline form B2 (25 mg, 0.05 mmol) was added water (0.4 mL, 16 vol.). The resultant suspension was stirred at room temperature for 4 hours. The solid was filtered with a sintered funnel (porosity 4), washed with water (3x0.2 mL) and dried under moderate vacuum at room temperature to give rivaroxaban hydrate form B1 as a white solid (20 mg, 88 % approximated yield).

### Example 11: Slurrying in water from rivaroxaban of formula (I) in the crystalline form E

To an assay tube equipped with magnetic stirrer containing rivaroxaban form E (20 mg, 0.04 mmol) was added water (0.2 mL, 10 vol.). The resultant suspension was stirred at room temperature for 3 hours. The solid was filtered with a sintered funnel (porosity 4), washed with water (2x0.1 mL) and dried under vacuum at room temperature to give rivaroxaban hydrate form B1 as a white solid (13 mg, 72% approximated yield).

¹H NMR (d6-DMSO, 400 MHz): δ = 8.96 (t, J = 6.0 Hz, 1 H); 7.68 (d, J = 3.9 Hz, 1 H); 7.58-7.52 (m, 2H); 7.43-7.37 (m, 2H); 7.18 (d, J = 3.9 Hz, 1 H); 4.88-4.79 (m, 1 H); 4.19 (s, 2H); 4.23-4.14 (m, 1 H); 4.00-3.93 (m, 2H); 3.85 (dd, J = 6.2 and 9.0 Hz, 1 H); 3.75-3.67 (m, 2H); 3.60 (t, J = 5.5 Hz, 2H).

IR (KBr): absorption bands at 3512 (s), 3084 (w), 2941 (w), 2876 (m), 2136 (w), 1758 (s), 1656 (m), 1151 (m), 993 (m), 922 (s), 827 (s) cm⁻¹.

DSC: Rivaroxaban hydrate form B1 is characterized for two endothermic intense peaks corresponding to the water loss at an onset 51.90 °C (fusion enthalpy -157.78 J/g) and the melting point at an onset 222.35 °C (fusion enthalpy -90.15 J/g), two exothermic sharp peaks corresponding to a polymorphic transformations at an onset 143.64 °C (formation enthalpy 9.30 J/g) and at an onset 188.35 °C (formation enthalpy 9.11 J/g), measured by DSC analysis (10 °C/min).

TGA: The TG analysis of rivaroxaban hydrate form B1 shows a weight loss at temperatures lower than the melting point. It corresponds to water loss (6.5%).

XRPD: The XRPD pattern of rivaroxaban hydrate form B1 is shown in FIG. 5. Table 3 shows the position (2theta angle (°)) and relative intensity (%) of the main peaks of the XRPD spectrum of FIG. 5.

**Table 3**

| Position [°2Th.] | Relative intensity [%] |
|---|---|
| 3.6 | 35 |
| 7.1 | 52 |
| 12.3 | 15 |
| 14.2 | 32 |
| 17.7 | 13 |
| 17.9 | 12 |
| 19.7 | 91 |
| 19.8 | 92 |
| 20.1 | 76 |
| 20.6 | 13 |
| 21.5 | 38 |
| 24.2 | 100 |
| 24.6 | 24 |
| 25.1 | 6 |
| 26.2 | 10 |
| 27.2 | 14 |
| 28.8 | 81 |
| 30.1 | 16 |
| 30.8 | 20 |
| 31.7 | 11 |
| 32.7 | 13 |
| 33.6 | 13 |
| 34.4 | 6 |
| 35.8 | 7 |
| 37.3 | 12 |
| 37.8 | 11 |
| 40.0 | 33 |
| 40.9 | 26 |
| 45.4 | 12 |
| 48.4 | 7 |

### Examples 12-14: Preparation of 5-chloro-N-{[(5S)-2-oxo-3-[4-(3-oxomorpholin-4-yl)phenyl]oxazolidin-5-yl]methyl}thiophene-2-carboxamide form III

### Example 12: Slurrying in water rivaroxaban of formula (I) in the crystalline form B2

To a round-bottom flask equipped with magnetic stirrer containing rivaroxaban hydrate B2 (3.01 g, 6.38 mmol) was added water (90 mL, 30 vol.). The resultant suspension was stirred at room temperature for 4 hours. The solid was filtered with a sintered funnel (porosity 4), washed with water (2x5 mL) and dried under vacuum at 40°C overnight to give rivaroxaban form III as a white solid (2.72 g, 98% approximated yield).

### Example 13: Slurrying in MeOH in the presence of concentrated HCl from rivaroxaban form I and wash with water

To a round-bottom flask equipped with magnetic stirrer containing rivaroxaban form I (50 mg, 0.11 mmol) was added methanol (750 µL, 15 vol.) and was stirred for a few minutes. Then, hydrochloric acid 37% (750 µL, 15 vol.) was added dropwise at 0°C. The resultant suspension was seeded with rivaroxaban of formula (I) in the crystalline form B2 and was stirred at room temperature for 1.5 hours. The solid was filtered with a sintered funnel (porosity 4), washed with water (2 x 0.2 mL) and dried under vacuum at 40°C overnight to give rivaroxaban form III as a white solid (42 mg, 84% yield).

### Example 14: Slurrying in water from rivaroxaban of formula (I) in the crystalline form E

To an assay tube equipped with magnetic stirrer containing rivaroxaban of formula (I) in the crystalline form E (101 mg, 0.20 mmol) was added water (1 mL, 10 vol.). The resultant suspension was stirred at room temperature for 3 hours. The solid was filtered with a sintered funnel (porosity 4), washed with water (2x0.5 mL) and dried under vacuum at 40°C overnight to give rivaroxaban form III as a white solid (79 mg, 91% yield).

### Examples 15-17: Preparation of 5-chloro-N-{[(5S)-2-oxo-3-[4-(3-oxomorpholin-4-yl)phenyl]oxazolidin-5-yl]methyl}thiophene-2-carboxamide form II

### Example 15: Slurrying in IPA rivaroxaban of formula (I) in the crystalline form B2

To a round-bottom flask equipped with magnetic stirrer containing rivaroxaban of formula (I) in the crystalline form B2 (100 mg, 0.21 mmol) was added isopropanol (1 mL, 10 vol.). The resultant suspension was stirred at - 10°C for 16 hours. The solid was filtered with a sintered funnel (porosity 4), washed with cold isopropanol (2x0.2 mL) and dried under vacuum at room temperature to give rivaroxaban form II as a white solid (60 mg, 66% approximated yield).

### Example 16: Slurrying in IPA rivaroxaban of formula (I) in the crystalline form B2

To a round-bottom flask equipped with magnetic stirrer containing rivaroxaban of formula (I) in the crystalline form B2 (1.00 g, 2.12 mmol) was added isopropanol (10 mL). The resultant suspension was seeded with rivaroxaban form II and was stirred at -10°C for 16 hours. The solid was filtered with a sintered funnel (porosity 3), washed with cold isopropanol (2x0.3 mL) and dried under vacuum at room temperature to give rivaroxaban form II as a white solid (886 mg, 96% approximated yield).

### Example 17: Slurrying in IPA from rivaroxaban of formula (I) in the crystalline form E

To an assay tube equipped with magnetic stirrer containing rivaroxaban of formula (I) in the crystalline form E (21 mg, 0.04 mmol) was added isopropanol (0.4 mL, 19 vol.). The resultant suspension was stirred at room temperature for 22 hours. The solid was filtered with a sintered funnel (porosity 4), washed with isopropanol (0.2 mL) and dried under vacuum at room temperature to give rivaroxaban form II as a white solid (15 mg, 82% yield).

### Example 18: Slurrying in IPA from rivaroxaban of formula (I) in the crystalline form E

To an assay tube equipped with magnetic stirrer containing rivaroxaban of formula (I) in the crystalline form E (100 mg, 0.20 mmol) was added isopropanol (1.5 mL, 15 vol.). The resultant suspension was seeded with rivaroxaban form II and was stirred at room temperature for 3 hours. The solid was filtered with a sintered funnel (porosity 4), washed with isopropanol (0.5 mL) and dried under vacuum at room temperature to give rivaroxaban form 11 as a white solid (83 mg, 95% yield).

### REFERENCES CITED IN THE APPLICATION

- WO 01/47919
- WO 2007/039132
- WO 2010/075631

## Claims

1. A process for the preparation of rivaroxaban of formula (I) 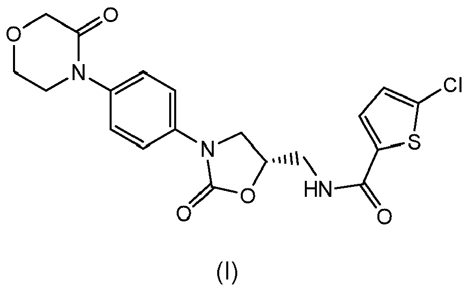 in the crystalline form II comprising:
a) slurrying rivaroxaban of formula (I) in a crystalline form selected from the crystalline form B2 and the crystalline form E in isopropanol; and
b) isolating rivaroxaban of formula (I) in the crystalline form II;
wherein:
the crystalline form II has an X-ray powder diffraction pattern comprising 2θ angle values at 12.8, 17.7, 18.1, 18.4, 18.9, 19.9, 20.8, 21.6, 22.0, 22.9, 24.0, 26.0, 26.4, 26.6, 27.1, 27.5, 31.0, and 31.5 ± 0.1 measured in an X-ray diffractometer with Cu Kα radiation (1.5418 A);
the crystalline form B2 has an X-ray powder diffraction pattern comprising 2θ angle values at 7.1, 14.2, 17.5, 19.8, 20.0, 21.4, 24.1, 24.5, 27.0, 28.7, 30.6, 39.8, 40.9, and 45.1 ± 0.1 measured in an X-ray diffractometer with Cu Kα radiation (1.5418 A); and
the crystalline form E has an X-ray powder diffraction pattern comprising 2θ angle values at 14.5, 18.2, 19.2, 20.2, 20.8, 21.9, 24.7, 28.1, and 31.9 ± 0.1 measured in an X-ray diffractometer with Cu Kα radiation (1.5418 A).

2. The process according to claim 1, further comprising the previous steps of:
(i) slurrying rivaroxaban of formula (I) in the crystalline form I in a mixture of an organic solvent and an acid other than HNO₃ to yield rivaroxaban of formula (I) in the crystalline form B2; or alternatively
slurrying rivaroxaban of formula (I) in the crystalline form I in a mixture of an organic solvent and HNO₃ to yield rivaroxaban of formula (I) in the crystalline form E; and
(ii) isolating the product obtained in step (i) to obtain rivaroxaban of formula (I) in the crystalline form B2 or in the crystalline form E respectively;
wherein the crystalline form I has an X-ray powder diffraction pattern comprising 2θ angle values at 9.0, 12.0, 14.3, 16.5, 17.4, 18.0, 19.5, 19.9, 21.7, 22.5, 23.3, 24.1, 24.7, 25.6, 26.6, 30.1, and 31.8 ± 0.1 measured in an X-ray diffractometer with Cu Kα radiation (1.5418 A).

3. The process according to claim 2, wherein the organic solvent of step (i) is a (C₁-C₆)alcohol or a (C₆-C₉)aromatic solvent.

4. The process according to claim 3, wherein the (C₁-C₆)alcohol is methanol.

5. The process according to claim 3, wherein the (C₆-C₉)aromatic solvent is toluene.

6. Rivaroxaban of formula (I) in the crystalline form B2, having an X-ray powder diffraction pattern comprising 2θ angle values at 7.1, 14.2, 17.5, 19.8, 20.0, 21.4, 24.1, 24.5, 27.0, 28.7, 30.6, 39.8, 40.9, and 45.1 ± 0.1 measured in an X-ray diffractometer with Cu Kα radiation (1.5418 A).

7. Rivaroxaban of formula (I) in the crystalline form E, having an X-ray powder diffraction pattern comprising 2θ angle values at 14.5, 18.2, 19.2, 20.2, 20.8, 21.9, 24.7, 28.1, and 31.9 ± 0.1 measured in an X-ray diffractometer with Cu Kα radiation (1.5418 A).

8. A process for the preparation of rivaroxaban of formula (I) in the crystalline form III comprising drying rivaroxaban of formula (I) in a crystalline form B1 at a temperature from 40°C to 100°C; wherein:
the crystalline form III has an X-ray powder diffraction pattern comprising 2θ angle values at 19.2, 19.7, 19.9, 23.3, 23.8, 24.3, 28.3, and 31.2 ± 0.1 measured in an X-ray diffractometer with Cu Kα radiation (1.5418 A); and
the crystalline form B1 has an X-ray powder diffraction pattern comprising 2θ angle values at 7.1, 14.2, 17.7, 19.7, 19.8, 20.1, 21.5, 24.2, 24.6, 27.2, 28.8, 40.0, 40.9, and 45.4 ± 0.1 measured in an X-ray diffractometer with Cu Kα radiation (1.5418 A).

9. The process according to claim 8, further comprising the previous steps of:
(a) slurrying rivaroxaban of formula (I) in a crystalline form selected from the crystalline form B2 and the crystalline form E in water; and
(b) isolating the product obtained in step (a) to obtain rivaroxaban of formula (I) in a crystalline form B1;
wherein the crystalline form B2 is as defined in claim 6 and the crystalline form E is as defined in claim 7.

10. The process according to claim 8, further comprising the previous step of washing rivaroxaban of formula (I) in the crystalline form B2 with water to obtain rivaroxaban of formula (I) in a crystalline form B1; wherein the crystalline form B2 is as defined in claim 6.

11. The process according to claim 9, further comprising the previous steps of:
(i) slurrying rivaroxaban of formula (I) in the crystalline form I in a mixture of an organic solvent and an acid other than HNO₃ to yield rivaroxaban of formula (I) in the crystalline form B2; or alternatively
slurrying rivaroxaban of formula (I) in the crystalline form I in a mixture of an organic solvent and HNO₃ to yield rivaroxaban of formula (I) in the crystalline form E; and
(ii) isolating the product obtained in step (i) to obtain rivaroxaban of formula (I) in the crystalline form B2 or in the crystalline form E respectively;
wherein the crystalline form I has an X-ray powder diffraction pattern comprising 2θ angle values at 9.0, 12.0, 14.3, 16.5, 17.4, 18.0, 19.5, 19.9, 21.7, 22.5, 23.3, 24.1, 24.7, 25.6, 26.6, 30.1, and 31.8 ± 0.1 measured in an X-ray diffractometer with Cu Kα radiation (1.5418 A).

12. Rivaroxaban of formula (I) in the crystalline form B1 having an X-ray powder diffraction pattern comprising 2θ angle values at 7.1, 14.2, 17.7, 19.7, 19.8, 20.1, 21.5, 24.2, 24.6, 27.2, 28.8, 40.0, 40.9, and 45.4 ± 0.1 measured in an X-ray diffractometer with Cu Kα radiation (1.5418 A).

13. A process for the preparation of rivaroxaban of formula (I) in the crystalline form B1 as defined in claim 12 comprising:
(a) slurrying rivaroxaban of formula (I) in a crystalline form selected from the crystalline form B2 and the crystalline form E in water; and
(b) isolating the product obtained in step a);
wherein the crystalline form B2 is as defined in claim 6 and the crystalline form E is as defined in claim 7.

14. A process for the preparation of rivaroxaban of formula (I) in the crystalline form B1 as defined in claim 12 comprising washing rivaroxaban of formula (I) in the crystalline form B2 with water; wherein the crystalline form B2 is as defined in claim 6.

15. The process according to claim 13, further comprising the previous steps of:
(i) slurrying rivaroxaban of formula (I) in the crystalline form I in a mixture of an organic solvent and an acid other than HNO₃ to yield rivaroxaban of formula (I) in the crystalline form B2; or alternatively
slurrying rivaroxaban of formula (I) in the crystalline form I in a mixture of an organic solvent and HNO₃ to yield rivaroxaban of formula (I) in the crystalline form E; and
(ii) isolating the product obtained in step (i) to obtain rivaroxaban of formula (I) in the crystalline form B2 or in the crystalline form E respectively; wherein the crystalline form I has an X-ray powder diffraction pattern comprising 2θ angle values at 9.0, 12.0, 14.3, 16.5, 17.4, 18.0, 19.5, 19.9, 21.7, 22.5, 23.3, 24.1, 24.7, 25.6, 26.6, 30.1, and 31.8 ± 0.1 measured in an X-ray diffractometer with Cu Kα radiation (1.5418 A).
